# EUROPEAN PATENT APPLICATION

(11) **EP 4 050 337 A1**
(43) Date of publication of application: **31.08.2022**
(21) Application number: 20878956.0
(22) Date of filing: 12.10.2020
(51) Int. Cl.: G01N 33/535, G01N 21/76, G01N 33/543

(54) **ENZYMATIC CHEMILUMINESCENCE IMMUNOASSAY METHOD FOR REDUCING BACKGROUND OF LUMINESCENT SUBSTRATE**

(30) Priority: 21.10.2019 CN 201911000587
(71) Applicant: Autobio Diagnostics Co., Ltd., Zhengzhou, Henan 450000 (CN)
(72) Inventor: MA, Jianjun, Zhengzhou, Henan 450000 (CN); LI, Shuangfa, Zhengzhou, Henan 450000 (CN); YU, Lin, Zhengzhou, Henan 450000 (CN); ZHENG, Yehuan, Zhengzhou, Henan 450000 (CN); FU, Guangyu, Zhengzhou, Henan 450000 (CN)
(74) Representative: Ipside
(86) International application number: PCT/CN2020/120330
(87) International publication number: WO 2021/078029

(57) **Abstract**

The present invention relates to the technical field of biological detection. Disclosed is an enzymatic chemiluminescence immunoassay method for reducing a background of a luminescent substrate. During enzymatic chemiluminescence immunoassay, an isolating liquid having the density of 0.75-0.98 kg/dm3 is added into a reaction vessel before the liquid A and the liquid B of the luminescent substrate of a chemiluminescent enzyme are in contact with each other and mixed; a luminescence signal value is read after a reaction. The immunoassay method of the present invention can avoid a background signal of the luminescent substrate from being affected by an oxidizing substance or a reducing substance in outside air, and has an extremely remarkable effect of reducing the fluctuation of the background signal of the luminescent substrate. Compared with a method without isolating air, the present invention greatly improves the sensitivity and the low-value precision of a kit, and has great significance for determining a clinical result.

## Description

### CROSS REFERENCE TO RELATED APPLICATIONS

This application claims the priority of Chinese Patent Application No. 201911000587.5, filed with China National Intellectual Property Administration on October 21, 2019, and titled with "ENZYMATIC CHEMILUMINESCENCE IMMUNOASSAY METHOD FOR REDUCING BACKGROUND OF LUMINESCENT SUBSTRATE".

### FIELD

The present invention belongs to the technical field of biological detection, specifically to a chemiluminescence enzyme immunoassay method for reducing the background from a luminescent substrate, in particular to a chemiluminescence enzyme immunoassay method for reducing the background of a magnetic particle chemiluminescence substrate.

### BACKGROUND

Chemiluminescence enzyme immunoassay (CLEIA) utilizes the catalysis of the labeled enzyme to make the luminescent agent (substrate) emit light. This type of luminescent agent that needs to be catalyzed by the enzyme to emit light is called an enzymatic reaction luminescent agent or a luminescent substrate. The luminescent substrates of enzymatic reactions refer to a class of luminescent substrates that emit light through the degradation by enzymes. Currently, the commonly used enzymes in chemiluminescence enzyme immunotechnology are horseradish peroxidase (HRP) and alkaline phosphatase (AP). The antigen or antibody is labeled with an enzyme such as horseradish peroxidase (HRP), and after immunoreaction with the corresponding antigen (antibody) in the sample to be tested, a complex of solid-phase coated with antibody-antigen -enzyme-labeled antibody is formed, after washing, adding the substrate (luminescent agent), the HRP enzyme catalyzes and decomposes the substrate to emit light, which is received by the optical quantum reading system, and the photomultiplier tube converts the light signals into electrical signals and amplifies the signals, and then the signals are sent to a computer data processing system to calculate the concentration of the analyte.

Most of HRP-based luminescent substrates are two-component (such as Autobio automatic luminescent substrates) systems, one of which is an oxidizing agent containing hydrogen peroxide, and the other is a reducing agent containing a luminescent agent luminol. Therefore, it is advisable to prepare two bottles of reagent solution separately and store them in two containers. The substrates are very stable and the validity period can reach about two years. HRP substrate systems usually include luminescence enhancers, such as certain phenolic reagents (such as o-iodophenol), phenolic compounds, aniline compounds, phenothiazine compounds, pyridine compounds, boric acid compounds, non-ionic detergents, etc. (see Chinese Patent Application CN103293145A), which can enhance the reaction of HRP-catalyzed luminol oxidation and prolong the luminescence time, and the luminescence sensitivity is very high. However, after adding a luminescence enhancer, although the luminescence sensitivity is very high, the background signal from the luminol substrate will fluctuate up and down, sometimes with a difference of tens to hundreds of times. The large variation of the background from substrate itself will cause great interference to the sensitivity and low-value precision of the kit.

### SUMMARY

In view of the above, the purpose of the present invention is to provide a method for reducing the background from the luminescent substrate itself, aiming at the problems existing in the prior art.

The inventor found through research that adding a luminescence enhancer will cause the entire luminescence system to be affected by air, especially the reducing or oxidizing substances in the luminescence system are affected by the air, which causes the background signal of the luminescence substrate to fluctuate up and down. Therefore, the present invention adopts the method of keeping the substances away from air during the reaction to solve the technical problem.

For realizing the purpose of the present invention, the present invention adopts following technical solution:

A chemiluminescence enzyme immunoassay method for reducing the background from a luminescent substrate. When performing a chemiluminescence enzyme immunoassay method, adding overlay liquid with a density of 0.75-0.98 kg/dm³ to a reaction container, contacting and mixing solution A and solution B of the luminescent substrate of the chemiluminescent enzyme, and reading the luminescence signal value after the reaction.

In the assay method of the present discourse, the overlay liquid has the characteristics of low density and chemical inertness. It is added before the solution A and solution B of the luminescent substrate are contacted and mixed, and coexists with the luminescent substrate in the container to form layers, which can isolate the luminescent substrate in the lower layer from contacting the air, therefore, the background signal of the luminescent substrate is prevented from being affected by oxidative or reducing substances in the outside air, and reducing fluctuations in the background signal of the luminescent substrate.

A person having ordinary skill in the art can understand that the specific operation of the assay method can be as follows: adding the solution A and solution B containing the luminescent substrates respectively to the container detecting the reaction through two pipelines of the machine, adding the overlay liquid to the container detecting the reaction through the third pipeline, and the machine immediately reads the luminescent signal value. For example, on the basis of the original two pipelines for adding the solution A and solution B of the substrate respectively to the Antubio automatic magnetic particle luminescence instrument A2000 or A2000PLUS, an additional pipeline is added for adding the overlay liquid.

In some embodiments, the chemiluminescent enzyme is horseradish peroxidase, and the solution A and solution B containing the luminescent substrates are respectively an oxidant containing hydrogen peroxide and a reducing agent containing luminol.

In some embodiments, the overlay liquid in the assay method of the present invention is light paraffin oil, light mineral oil or light white oil.

Further, preferably, the models of the light paraffin oil or light mineral oil are 3#, 5#, 7#, 10#, 15#, 26#, 32#, 46#, 68#, 90#, 100# or 150#.

Preferably, the model of the light white oil is D80, D85, D95, D100, D120 or D130.

It can be seen from the above-mentioned technical solutions that the present invention provides a chemiluminescence enzyme immunoassay method for reducing the background from a luminescent substrate. When performing a chemiluminescence enzyme immunoassay method, adding overlay liquid with a density of 0.75-0.98 kg/dm³ to a reaction container before contacting and mixing solution A and solution B of the luminescent substrate of the chemiluminescent enzyme, and reading the luminescence signal value after the reaction. The assay method of the present invention can prevent the background signal of the luminescent substrate from being affected by oxidizing or reducing substances in the outside air, and the effect of reducing the fluctuation of the background signal of the luminescent substrate is extremely significant. Compared with not isolating air, the sensitivity and low-value precision of the kit are greatly improved, which is of great significance to the judgment of clinical results.

### BRIEF DESCRIPTION OF DRAWINGS

In order to illustrate the embodiments of the present invention or the technical solutions in the prior art more clearly, the following briefly introduces the drawings need to be used in the description of the embodiments or the prior art.
Figure 1 shows the comparison of the signal values of the luminescent substrate at different times with or without adding 7# light paraffin oil in Example 1;
Figure 2 shows the effect of adding D80 light white oil in Example 3 on the detection sensitivity and linear range when using the luminescent substrate with horseradish peroxidase (with the relative concentration of the gradient enzyme as the abscissa);
Figure 3 shows the effect of adding D80 light white oil in Example 3 on the detection sensitivity and linear range when using the luminescent substrate with horseradish peroxidase (with the absolute concentration of the gradient enzyme as the abscissa).

### DETAILED DESCRIPTION

The present invention discloses a method for reducing the background from a luminescent substrate. A person having ordinary skill in the art can learn from the content of present document and appropriately improve the process parameters to achieve. It should be particularly pointed out that all similar substitutions and modifications are obvious to a person having ordinary skill in the art, and they are deemed to be included in the present invention. The methods and products of the present invention have been described through preferred embodiments, and it is obvious that relevant persons can make modifications or appropriate changes and combinations of the methods described herein without departing from the content, spirit and scope of the present invention to realize and apply the technology of the present invention.

In order to further understand the present invention, the technical solutions in the embodiments of the present invention will be clearly and completely described below with reference to the embodiments of the present invention. Obviously, the described embodiments are only a part of the embodiments of the present invention, rather than all the embodiments. Based on the embodiments of the present invention, all other embodiments obtained by a person having ordinary skill in the art without creative efforts shall fall within the protection scope of the present invention.

Unless otherwise specified, the reagents or instruments involved in the embodiments of the present invention are all commercially available products, which can be purchased through commercial channels.

### Examples 1

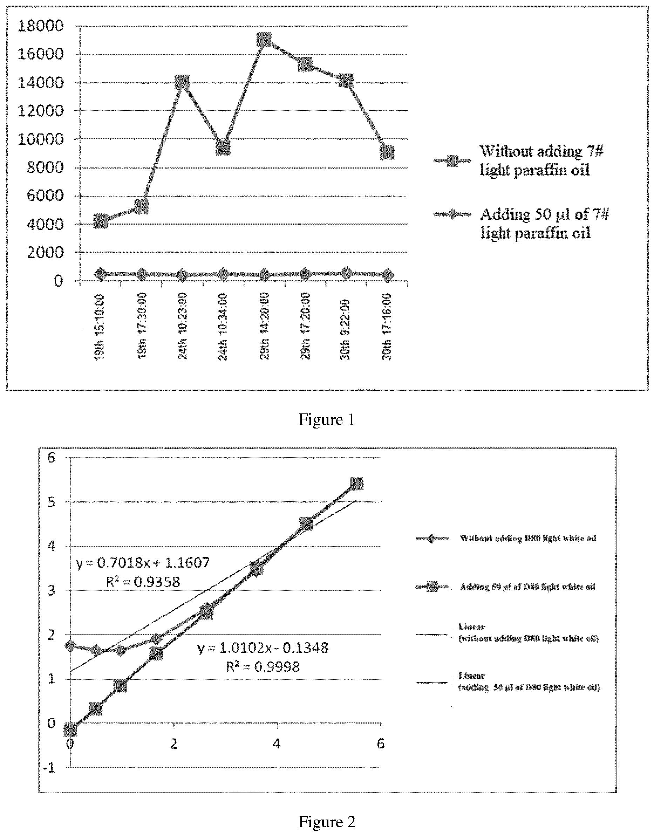

The background signal value of the luminescent substrate was measured at different times for 8 times during four days. Each time, 10 wells of a 96-well luminescent plate were used for experiment and 50 µl of each of solution A and B of the substrate kit (AntuBio Automatic Immunoassay System) was added into each well, respectively. 50 µl of 7# light paraffin oil was added to 5 wells but not the other 5 wells. The luminescence signal read of 10 wells was immediately measured, and the mean value of 5 wells was taken respectively, as shown in Table 1 and Figure 1.

**Table 1 Comparison of the signal reads from the luminescent substrate at different time points with or without adding 7# light paraffin oil**

| Date of Detection | Time of Detection | Luminescence Signal Read with 7# Light Paraffin Oil Added | Luminescence Signal Read without 7# Light Paraffin Oil Added |
|---|---|---|---|
| 19^{th} | 15:10 | 491 | 3720 |
| | 17:30 | 486 | 4771 |
| 24^{th} | 10:23 | 399 | 13635 |
| | 10:34 | 482 | 8931 |
| 29^{th} | 14:20 | 426 | 16632 |
| | 17:20 | 436 | 14834 |
| 30^{th} | 9:22 | 499 | 13622 |
| | 17:16 | 429 | 8683 |
| Mean Value | | 456.0 | 10603.5 |
| Coefficient of Variation (CV) | | 8.3% | 45.1% |

Note: detection instrument for each detection is Antu A2000PLUS (JC-147), and the luminescence signal read of each group is the mean value of 5 wells.

The results show that the average value of the background from the substrate of each well with 7# light paraffin oil added at different times was below 500, and the coefficient of variation was 8.3%. In the contract, the background luminescence signal from the substrate of each well without 7# light paraffin oil added was higher and unstable, and the luminescence signal value varied greatly from 3720 to 16632, the coefficient of variation reached 45.1%, and the precision of the signal value in the low value area was poor.

### Example 2. Influence of adding 3# light mineral oil on the luminescence signal value when using four detection kits

Antu automatic magnetic particle chemiluminescence instrument A2000PLUS (JC-147) was used to detect the signals from thyroid stimulating hormone (TSH) detection kit, parathyroid hormone (PTH) detection kit, cardiac troponin I (CTNI) detection kit, hepatitis B virus surface antigen (HBsAg) detection kit (purchased from Antu) using different luminescent substrates, respectively. In a 96-well reflective plate, 10 wells were used for blank control or standard in each kit. 50 µl of each of luminescent substrate A and B was added. 50 µl of 3# light mineral oil was added to 5 wells for covering, but was not added to the other 5 wells. The luminescence signal read of 10 wells was measured, and the mean value of 5 wells was calculated respectively, as shown in Table 2.

**Table 2 The effect of adding 3# light mineral oil on the luminescence signal reads using four detection kits**

| Test | Blank control 0 and Standard | Signal Read | | Signal to Noise Ratio | | Ratio of Signal with/without covering |
|---|---|---|---|---|---|---|
| | | Covering | Without Covering | Covering | Without Covering | |
| TSH (µIU/mL) | S0 (0.00) | 2767 | 12941 | 243.7 | 50.3 | 0.21 |
| | S1 (0.20) | 674444 | 650510 | | | 1.04 |
| | S2 (0.50) | 1874322 | 1853129 | | | 1.01 |
| | S3 (2.00) | 8575853 | 8646160 | | | 0.99 |
| | S4 (10.00) | 50598940 | 52508041 | | | 0.96 |
| | S5 (50.00) | 206549476 | 208938289 | | | 0.99 |
| | S6 (100.00) | 342293207 | 340627599 | | | 1.00 |
| PTH (pg/mL) | S0 (0) | 1044 | 21829 | 7.8 | 1.7 | 0.05 |
| | S1 (10) | 8140 | 37232 | | | 0.22 |
| | S2 (50) | 51159 | 87974 | | | 0.58 |
| | S3 (250) | 644285 | 699794 | | | 0.92 |
| | S4 (1000) | 4273526 | 4674197 | | | 0.91 |
| | S5 (3000) | 12931636 | 15149477 | | | 0.85 |
| CTNI (µg/mL) | S0 (0) | 6149 | 20750 | 25.2 | 7.7 | 0.30 |
| | S1 (0.30) | 155043 | 158978 | | | 0.98 |
| | S2 (1.20) | 1146923 | 956272 | | | 1.20 |
| | S3 (5.00) | 9262408 | 8844597 | | | 1.05 |
| | S4 (25.00) | 108567819 | 109355280 | | | 0.99 |
| | S5 (100.00) | 418265303 | 483149657 | | | 0.87 |
| HBsAg (IU/mL) | S0 (0) | 7322 | 23046 | 16.3 | 6.6 | 0.32 |
| | S1 (0.05) | 119283 | 151612 | | | 0.79 |
| | S2 (0.5) | 924757 | 1111483 | | | 0.83 |
| | S3 (5.0) | 12323086 | 14098394 | | | 0.87 |
| | S4 (50.0) | 113141353 | 124834536 | | | 0.91 |
| | S5 (250.0) | 384798282 | 434212011 | | | 0.89 |

Note: The luminescence signal value of each group is the mean value of 5 wells, and the corresponding concentrations are shown in parentheses.

The results show that comparing the luminescence signal value of each detection well S0 without 3# light mineral oil added, the signal of the standard S0 with 3# light mineral oil added was significantly lower, which were 21%, 5%, 30%, and 32% of the corresponding well without covering with oil in four tests, respectively. The addition of 3# light mineral oil had little effect on the luminescence signal value of the series of standard in the kit. After adding 3# light mineral oil to the wells in different tests, each of the signal to noise ratio, for example, S1/S0 was significantly improved, indicating that the influence of air on the reagent was avoided after the 3# light mineral oil was added to cover the reaction system, and the luminescence signal value recorded was the real signal value from the reagent to be tested.

### Example 3. Influence of adding D80 light white oil on the detection sensitivity and linear range of the reaction between luminescent substrate and horseradish peroxidase

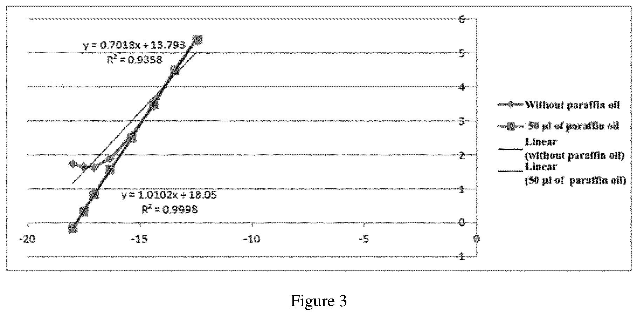

HRP working enzyme in AntuBio's Hepatitis B Virus Surface Antigen (HBsAg) Magnetic Particle Detection Kit (10 ml/100 people, containing HRP-labeled antibody, protein protectant, stabilizer, etc.) was used, and 8 concentrations were prepared by serial dilution (50µl of enzyme added to 100µl of distilled water) with purified water (e.g., volume ratio 1:3). The 8^{th} maximum dilution HRP enzyme concentration was set to 1 (its actual concentration was 0.0000000000000000001mol/L, i.e., 1E-18mol/L), then the gradient enzyme concentration was defined as 1.00 (the actual concentration was 1E-18mol/L), 3.00 (the actual concentration was 3E-18mol/L), 9.00 (the actual concentration was 9E-18mol/L), 45.00 (the actual concentration was 4.5E-17mol/L), 418.50 (the actual concentration was 4.19E-16mol/L) , 3892.05 (the actual concentration was 3.89E-15mol/L), 36196.07 (the actual concentration was 3.62E-14mol/L), 336623.40 (the actual concentration was 3.37E-13mol/L). 10 µl enzyme of each concentration in the gradient serial was added to each well of a 96-well microplate, then, 50 µl of each of substrate A and B was added. And 50 µl of D80 light white oil was added to half number of the wells, but was not added to the other half number of the wells. The luminescence signal was immediately measured and shown in Table 3, and the log-log between the gradient enzyme concentration and the luminescence signal read was plotted, as shown in Figures 2 and 3.

**Table 3 The effect of adding D80 light white oil on the detection signal of the reaction of luminescent substrate with gradient concentration of horseradish peroxidase**

| Enzyme Gradients (HRP) | Signal without Covering | Signal with Covering | Enzyme Gradients (log) | Without Covering (log) | Covering (log) |
|---|---|---|---|---|---|
| 1.00 | 54.95 | 0.69 | 0.00 (-18) | 1.74 | -0.16 |
| 3.00 | 44.85 | 2.15 | 0.48 (-17.52287875) | 1.65 | 0.33 |
| 9.00 | 43.35 | 7.03 | 0.95 (-17.04575749) | 1.64 | 0.85 |
| 45.00 | 79.70 | 36.79 | 1.65 (-16.34678749) | 1.90 | 1.57 |
| 418.50 | 393.17 | 313.64 | 2.62 (-15.37830454) | 2.59 | 2.50 |
| 3892.05 | 2800.41 | 3230.93 | 3.59 (-14.40982159) | 3.45 | 3.51 |
| 36196.07 | 32768.71 | 32459.47 | 4.56 (-13.44133858) | 4.52 | 4.51 |
| 336623.40 | 251593.93 | 252172.04 | 5.53 (-12.4728557) | 5.40 | 5.40 |

Among them, in the column of enzyme gradient (log), the value before the parentheses is the log value of the relative concentration of the enzyme, and the value in the parentheses is the log value of the absolute concentration of the enzyme.

The results show that, when perform test using gradient concentration of HRP, adding D80 light white oil luminescent substrate greatly improved the detection sensitivity of horseradish peroxidase HRP by 3⁴(81) times, and the linear range was wider. Low concentrations of horseradish peroxidase could also be detected in the gradient enzyme shown in the Table 3 (R² = 0.9998 for signal values in the concentration range of 1-336623.40). Therefore, the detection sensitivity to luminescent substrates using the overlay liquid was higher, and the detection linear range was wider. In the magnetic particle chemiluminescence detection technology, the application of overlay liquid is of great significance.

## Claims

1. A chemiluminescence enzyme immunoassay method for reducing the background from a luminescent substrate, comprising during a chemiluminescence enzyme immunoassay, adding an overlay liquid with a density of 0.75-0.98 kg/dm³ to a reaction container, contacting and mixing solution A and solution B containing the luminescent substrate of the chemiluminescent enzyme, and recording a luminescence signal after the reaction.

2. The method according to claim 1, wherein the chemiluminescent enzyme is horseradish peroxidase, and the solution A and solution B are respectively an oxidant comprising hydrogen peroxide and a reducing agent comprising luminol.

3. The method according to claim 1, wherein the overlay liquid is a light paraffin oil, a light mineral oil or a light white oil.

4. The method according to claim 3, wherein the model of the light paraffin oil or light mineral oil is 3#, 5#, 7#, 10#, 15#, 26#, 32#, 46#, 68#, 90#, 100# or 150#.

5. The method according to claim 3, wherein the model of the light white oil is D80, D85, D95, D100, D120 or D130.
